# EUROPEAN PATENT APPLICATION

(11) **EP 3 932 304 A1**
(43) Date of publication of application: **05.01.2022**
(21) Application number: 21191121.9
(22) Date of filing: 21.12.2015
(51) Int. Cl.: A61B 5/0538, A61B 18/14

(54) **BALLOON FOR ABLATION AROUND PULMONARY VEINS**

(30) Priority: 22.12.2014 US 201414578807
(62) Divisional of application: 15201723.2
(71) Applicant: Biosense Webster (Israel) Ltd., 2066717 Yokneam (IL)
(72) Inventor: GOVARI, Assaf, 2066717 Yokneam (IL); BEECKLER, Christopher Thomas, Irwindale, 91706 (US); KEYES, Joseph Thomas, Irwindale, 91706 (US); HETTEL, Rowan Olund, Irwindale, 91706 (US)
(74) Representative: Carpmaels & Ransford LLP

(57) **Abstract**

Cardiac ablation is carried out by introducing a catheter into the left atrium, extending a lasso guide through the lumen of the catheter to engage the wall of a pulmonary vein, and deploying a balloon over the lasso guide. The balloon has an electrode assembly disposed its exterior. The electrode assembly includes a plurality of ablation electrodes circumferentially arranged about the longitudinal axis of the catheter. The inflated balloon is positioned against the pulmonary vein ostium, so that the ablation electrodes are in galvanic contact with the pulmonary vein, and electrical energy is conducted through the ablation electrodes to produce a circumferential lesion that circumscribes the pulmonary vein.

## Description

### BACKGROUND OF THE INVENTION

### 1. Field of the Invention.

This invention relates to medical devices. More particularly, this invention relates to improvements in cardiac catheterization.

### 2. Description of the Related Art.

Cardiac arrhythmias, such as atrial fibrillation, occur when regions of cardiac tissue abnormally conduct electric signals to adjacent tissue, thereby disrupting the normal cardiac cycle and causing asynchronous rhythm.

Procedures for treating arrhythmia include surgically disrupting the origin of the signals causing the arrhythmia, as well as disrupting the conducting pathway for such signals. By selectively ablating cardiac tissue by application of energy via a catheter, it is sometimes possible to cease or modify the propagation of unwanted electrical signals from one portion of the heart to another. The ablation process destroys the unwanted electrical pathways by formation of non-conducting lesions.

Circumferential lesions at or near the ostia of the pulmonary veins have been created to treat atrial arrhythmias. U.S. Patent Nos. 6,012,457 and 6,024,740, both to Lesh, disclose a radially expandable ablation device, which includes a radiofrequency electrode. Using this device, it is proposed to deliver radiofrequency energy to the pulmonary veins in order to establish a circumferential conduction block, thereby electrically isolating the pulmonary veins from the left atrium.

U.S. Patent No. 6,814,733 to Schwartz et al., which is commonly assigned herewith and herein incorporated by reference, describes a catheter introduction apparatus having a radially expandable helical coil as a radiofrequency emitter. In one application the emitter is introduced percutaneously, and transseptally advanced to the ostium of a pulmonary vein. The emitter is radially expanded, which can be accomplished by inflating an anchoring balloon about which the emitter is wrapped, in order to cause the emitter to make circumferential contact with the inner wall of the pulmonary vein. The coil is energized by a radiofrequency generator, and a circumferential ablation lesion is produced in the myocardial sleeve of the pulmonary vein, which effectively blocks electrical propagation between the pulmonary vein and the left atrium.

Another example is found in U.S. Patent No. 7,340,307 to Maguire, et al., which proposes a tissue ablation system and method that treats atrial arrhythmia by ablating a circumferential region of tissue at a location where a pulmonary vein extends from an atrium. The system includes a circumferential ablation member with an ablation element and includes a delivery assembly for delivering the ablation member to the location. The circumferential ablation member is generally adjustable between different configurations to allow both the delivery through a delivery sheath into the atrium and the ablative coupling between the ablation element and the circumferential region of tissue.

### SUMMARY OF THE INVENTION

Embodiments of the present invention provide a catheter that enables delivery of an ablation balloon to the ostium of a pulmonary vein. The balloon and the method of delivery simplify the procedure for the physician.

There is provided according to embodiments of the invention a method of ablation, which is carried out by introducing a catheter into a left atrium of a heart, extending a lasso guide through the lumen of the catheter to engage an interior wall of a pulmonary vein, deploying an inflated balloon over a portion of the lasso guide, the balloon having an electrode assembly disposed on an exterior wall thereof. The electrode assembly includes a plurality of ablation electrodes circumferentially arranged about the longitudinal axis. The method is further carried out by positioning the balloon against the pulmonary vein ostium, so that the ablation electrodes are in galvanic contact with the pulmonary vein, and conducting electrical energy through the ablation electrodes to produce a circumferential lesion that circumscribes the pulmonary vein.

One aspect of the method includes injecting a contrast agent through the catheter into the pulmonary vein after inflating and positioning the balloon.

A further aspect of the method includes injecting a contrast agent through the catheter into the balloon after positioning the balloon.

In still another aspect of the method, the lasso guide has a mapping electrode disposed thereon. The method is further carried out by obtaining a pre-ablation electrogram with the mapping electrode prior to performing conducting electrical energy through the ablation electrodes.

In another aspect of the method, the lasso guide has a mapping electrode disposed thereon. The method is further carried out by obtaining a post-ablation electrogram with the mapping electrode after performing conducting electrical energy through the ablation electrodes.

There is further provided according to embodiments of the invention an ablation apparatus including a probe, a lasso guide that assumes a collapsed state for delivery through the lumen of the probe and assumes an expanded state after delivery through the probe. The lasso guide has a plurality of mapping electrodes that are connectable to electrocardiographic circuitry. The apparatus further includes an inflatable balloon deployable through the lumen over the lasso guide, the balloon having a plurality of ablation electrodes arranged circumferentially about the longitudinal axis on its exterior wall. The balloon is fenestrated by a plurality of irrigation pores and is connected to a source of fluid for passage of the fluid through the pores.

In an additional aspect of the apparatus, a subassembly has a plurality of strips radiating outwardly from the longitudinal axis of the balloon, wherein the ablation electrodes are disposed on the strips.

According to another aspect of the apparatus, the subassembly has apertures formed therethrough that are in fluid communication with the pores of the balloon.

In another aspect of the apparatus wires in the distal portion of the probe lead to the ablation electrodes, and the strips of the subassembly comprise pigtails extending over a surface of the balloon and overlying respective wires.

### BRIEF DESCRIPTION OF THE SEVERAL VIEWS OF THE DRAWINGS

For a better understanding of the present invention, reference is made to the detailed description of the invention, by way of example, which is to be read in conjunction with the following drawings, wherein like elements are given like reference numerals, and wherein:
Fig. 1 is a pictorial illustration of a system for performing catheterization procedures on a heart, in accordance with a disclosed embodiment of the invention;
Fig. 2 is a view of the distal portion of the catheter shown in Fig. 1 in accordance with an embodiment of the invention;
Fig. 3 is another view of the distal portion of the catheter shown in Fig. 1 in accordance with an embodiment of the invention;
Fig. 4 is a view of the distal portion of the catheter shown in Fig. 1 in an operating position for ablation in accordance with an embodiment of the invention;
Fig. 5 is a bottom plan view of the catheter electrode assembly shown in Fig. 4 in accordance with an embodiment of the invention;
Fig. 6 is a top plan view of the catheter electrode assembly shown in Fig. 4 in accordance with an embodiment of the invention;
Fig. 7 is a side elevation of an embodiment of a balloon of the catheter shown in Fig. 4 in accordance with an embodiment of the invention;
Fig. 8 is a cut-away sectional view through line 8-8 of the balloon shown in fig. 7 in accordance with an embodiment of the invention; and
Fig. 9 is a flow-chart of a method of pulmonary vein isolation in accordance with an embodiment of the invention.

### DETAILED DESCRIPTION OF THE INVENTION

In the following description, numerous specific details are set forth in order to provide a thorough understanding of the various principles of the present invention. It will be apparent to one skilled in the art, however, that not all these details are necessarily needed for practicing the present invention. In this instance, well-known circuits, control logic, and the details of computer program instructions for conventional algorithms and processes have not been shown in detail in order not to obscure the general concepts unnecessarily.

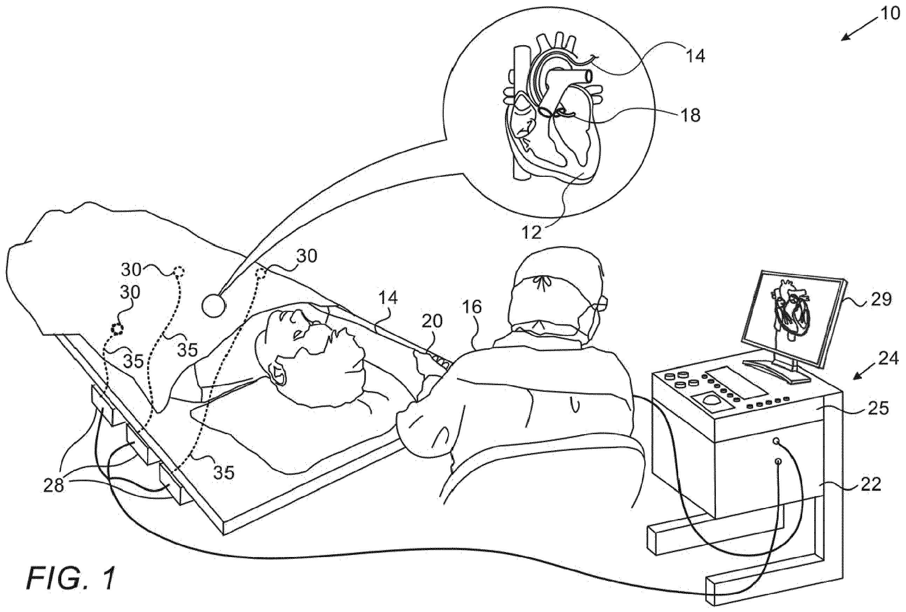

Turning now to the drawings, reference is initially made to Fig. 1, which is a pictorial illustration of a system 10 for evaluating electrical activity and performing ablative procedures on a heart 12 of a living subject, which is constructed and operative in accordance with a disclosed embodiment of the invention. The system comprises a catheter 14, which is percutaneously inserted by an operator 16 through the patient's vascular system into a chamber or vascular structure of the heart 12. The operator 16, who is typically a physician, brings the catheter's distal tip 18 into contact with the heart wall, for example, at an ablation target site. Electrical activation maps may be prepared, according to the methods disclosed in U.S. Patent Nos. 6,226,542, and 6,301,496, and in commonly assigned U.S. Patent No. 6,892,091, whose disclosures are herein incorporated by reference. One commercial product embodying elements of the system 10 is available as the CARTO^{®} 3 System, available from Biosense Webster, Inc., 3333 Diamond Canyon Road, Diamond Bar, CA 91765. This system may be modified by those skilled in the art to embody the principles of the invention described herein.

Areas determined to be abnormal, for example by evaluation of the electrical activation maps, can be ablated by application of thermal energy, e.g., by passage of radiofrequency electrical current through wires in the catheter to one or more electrodes at the distal tip 18, which apply the radiofrequency energy to the myocardium. The energy is absorbed in the tissue, heating it to a point (typically above 60°C) at which it permanently loses its electrical excitability. When successful, this procedure creates non-conducting lesions in the cardiac tissue, which disrupt the abnormal electrical pathway causing the arrhythmia. The principles of the invention can be applied to different heart chambers to diagnose and treat many different cardiac arrhythmias.

The catheter 14 typically comprises a handle 20, having suitable controls on the handle to enable the operator 16 to steer, position and orient the distal end of the catheter as desired for the ablation. To aid the operator 16, the distal portion of the catheter 14 contains position sensors (not shown) that provide signals to a processor 22, located in a console 24. The processor 22 may fulfill several processing functions as described below.

Wire connections 35 link the console 24 with body surface electrodes 30 and other components of a positioning sub-system for measuring location and orientation coordinates of the catheter 14. The processor 22 or another processor (not shown) may be an element of the positioning subsystem. Catheter electrodes (not shown) and the body surface electrodes 30 may be used to measure tissue impedance at the ablation site as taught in U.S. Patent No. 7,536,218, issued to Govari et al., which is herein incorporated by reference. Temperature sensors (not shown), typically a thermocouple or thermistor, may be mounted on ablation surfaces on the distal portion of the catheter 14 as described below.

The console 24 typically contains one or more ablation power generators 25. The catheter 14 may be adapted to conduct ablative energy to the heart using any known ablation technique, e.g., radiofrequency energy, ultrasound energy, and laser-produced light energy. Such methods are disclosed in commonly assigned U.S. Patent Nos. 6,814,733, 6,997,924, and 7,156,816, which are herein incorporated by reference.

In one embodiment, the positioning subsystem comprises a magnetic position tracking arrangement that determines the position and orientation of the catheter 14 by generating magnetic fields in a predefined working volume and sensing these fields at the catheter, using field generating coils 28. The positioning subsystem is described in U.S. Patent No. 7,756,576, which is hereby incorporated by reference, and in the above-noted U.S. Patent No. 7,536,218.

As noted above, the catheter 14 is coupled to the console 24, which enables the operator 16 to observe and regulate the functions of the catheter 14. Console 24 includes a processor, preferably a computer with appropriate signal processing circuits. The processor is coupled to drive a monitor 29. The signal processing circuits typically receive, amplify, filter and digitize signals from the catheter 14, including signals generated by sensors such as electrical, temperature and contact force sensors, and a plurality of location sensing electrodes (not shown) located distally in the catheter 14. The digitized signals are received and used by the console 24 and the positioning system to compute the position and orientation of the catheter 14, and to analyze the electrical signals from the electrodes.

In order to generate electroanatomic maps, the processor 22 typically comprises an electroanatomic map generator, an image registration program, an image or data analysis program and a graphical user interface configured to present graphical information on the monitor 29.

Typically, the system 10 includes other elements, which are not shown in the figures for the sake of simplicity. For example, the system 10 may include an electrocardiogram (ECG) monitor, coupled to receive signals from one or more body surface electrodes, in order to provide an ECG synchronization signal to the console 24. As mentioned above, the system 10 typically also includes a reference position sensor, either on an externally-applied reference patch attached to the exterior of the subject's body, or on an internally-placed catheter, which is inserted into the heart 12 maintained in a fixed position relative to the heart 12. Conventional pumps and lines for circulating liquids through the catheter 14 for cooling the ablation site are provided. The system 10 may receive image data from an external imaging modality, such as an MR1 unit or the like and includes image processors that can be incorporated in or invoked by the processor 22 for generating and displaying images.

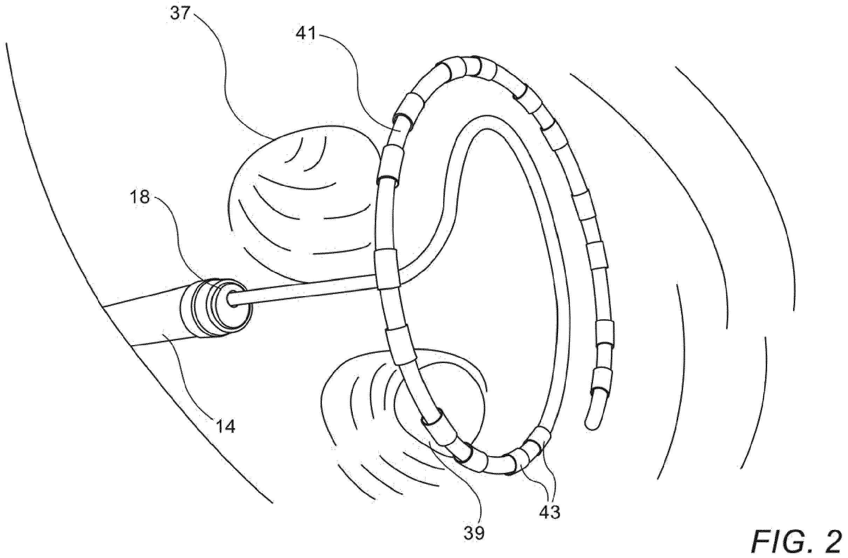

Reference is now made to Fig. 2, which is a view of the distal portion of the catheter 14 (Fig. 1) in accordance with an embodiment of the invention. The distal tip 18 of the catheter is within the left atrium of the heart 12 (Fig. 1). Pulmonary vein ostia 37, 39 are visible. A lasso guide 41 has been partially deployed beyond the distal tip 18. The lasso guide 41 may have a shape memory, and when extended through the distal tip 18 of the catheter 14, the distal portion of the lasso guide 41 configures itself into a ring or spiral. Multiple ring electrodes 43 may be disposed on the lasso guide 41. The electrodes 43 are useful for obtaining electrograms to confirm electrical isolation of the pulmonary vein following ablation while the lasso guide 41 is still engaged with the wall of the pulmonary vein. Other types of electrodes and sensors may be mounted on the lasso guide 41, for example contact force sensors and magnetic location sensors.

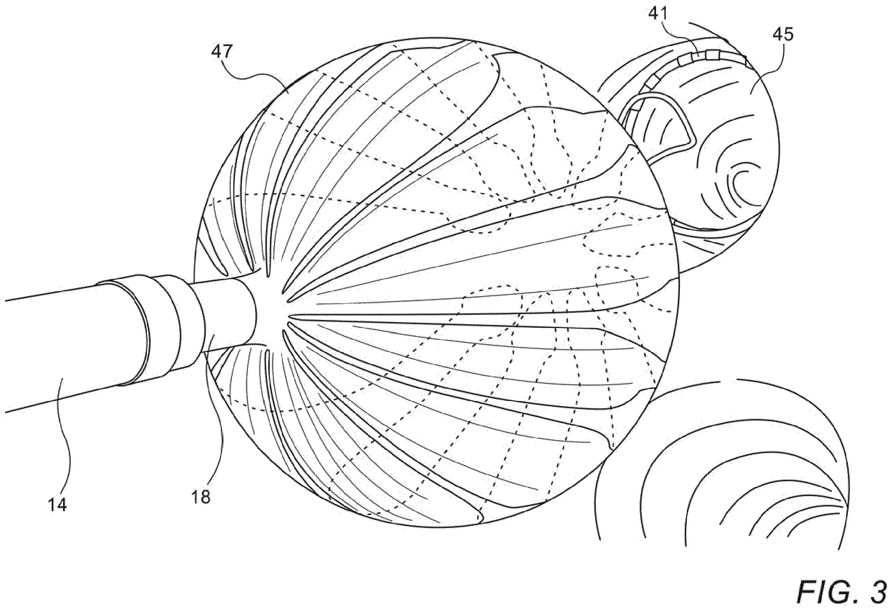

Reference is now made to Fig. 3, which is a view of the distal portion of the catheter 14 (Fig. 1) in accordance with an embodiment of the invention. The lasso guide 41 has been deployed and is engaged with the wall of pulmonary vein 45. A balloon 47 has been inflated, aided by the stability provided by the lasso guide 41 that is anchored against the vessel wall. Correct placement of the balloon 47 can be verified by injecting a contrast agent through the catheter 14. Additionally or alternatively the contrast agent may be injected into the balloon 47.

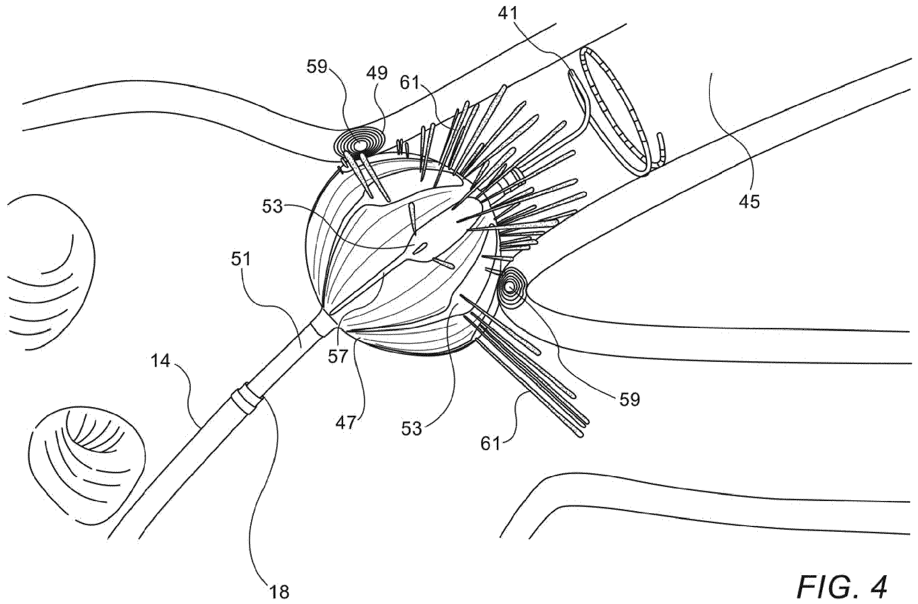

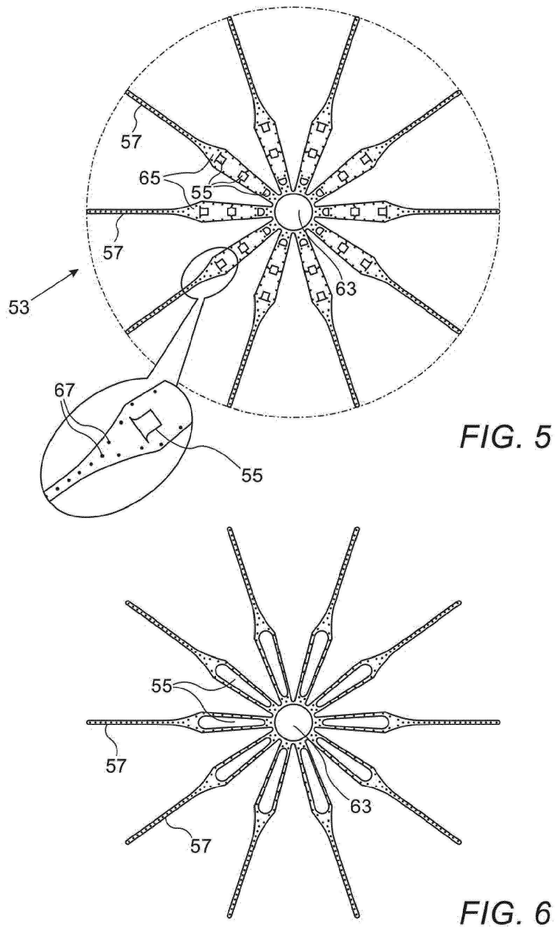

Reference is now made to Fig. 4, which is a pictorial side view of distal segment of the catheter 14 (Fig. 1) shown in an operating position at ostium 49 of pulmonary vein 45 in accordance with an embodiment of the invention. The lasso guide 41 has been fully extended through the distal tip 18. Once the guide is positioned in the vein, the balloon 47, which is mounted on a shaft 51, extends beyond the distal tip 18 of the catheter 14. The balloon 47 is inflated by injection with saline solution, in order to close off the vein at the ostium 49. The balloon 47 is fenestrated. Apertures or pores (best seen in Fig. 6) allow the saline to irrigate the ostium 49. The balloon 47 has an electrode assembly 53 disposed on its eternal surface. Multiple ablation electrodes are disposed on the electrode assembly 53, as best seen in Fig. 5. The components of the electrode assembly 53 are elongate, and directed longitudinally in respective planes that are normal to the shaft 51 in order to maximize galvanic contact between its electrodes 55 (Fig. 5) and the wall of the ostium 49. Pigtails 57 prevent the electrode assembly 53 from delaminating when the balloon 47 is retracted into the shaft of the catheter 14 and protect wires (not shown) leading to the electrodes of the electrode assembly 53. Other geometric configurations for the electrode assembly 53 are possible, for example a spiral arrangement, or concentric rings. Passage of electrical energy through the electrodes 55 (Fig. 5) creates a circumferential lesion 59 at the ostium 49 that blocks electrical propagation and isolates the pulmonary vein from the heart. The ablation site is cooled by flow of a cooling fluid 61 through pores formed in the balloon 47 and the electrode assembly 53. Alternatively, a portion of the electrodes 55 may be configured for electrical mapping.

Reference is now made to Fig. 5, which is a bottom plan view of the electrode assembly 53 in accordance with an embodiment of the invention. The electrode assembly 53 is shown detached from the balloon 47. The bottom surface of the electrode assembly 53 is adapted to be adhered to the external surface of the balloon 47 (Fig. 4) The electrode assembly 53 comprises a central aperture 63 through which the shaft 51 (Fig. 4) extends. This arrangement permits injection of contrast material or sampling through the shaft 51 as may be required by the medical procedure. The electrode assembly 53 comprises a substrate of radiating strips 65 that extend about the balloon 47 and are brought into contact with a pulmonary vein ostium when the balloon is inflated and navigated to the pulmonary vein. Electrodes 55 are disposed on each of the strips 65, and come into galvanic contact with the ostium during an ablation operation, during which electrical current flows through the electrodes 55 and the ostium. Ten strips 65 are shown in the example of Fig. 5 and are evenly distributed about of central axis the aperture 63. Other numbers of strips are possible. However, there should be a sufficiently small angle between adjacent strips 65 such that at least one continuous circumferential lesion is produced in the pulmonary vein when the electrodes 55 are activated for ablation.

Numerous pores 67 (typically 25-100 microns in diameter) are formed through each of the strips 65 and perforate the underlying balloon 47 as well. The pores 67 conduct a flow of cooling irrigation fluid from the interior of the balloon 47 onto and near the ablation site. The flow rate may be varied by a pump control (not shown) from an idle rate of about 4mL/min to the ablation flow rate of 60mL/min.

Reference is now made to Fig. 6, which is a top plan view of the electrode assembly 53 in accordance with an embodiment of the invention. Electrodes 55 are shown. In operation they come into contact with the wall of the pulmonary vein.

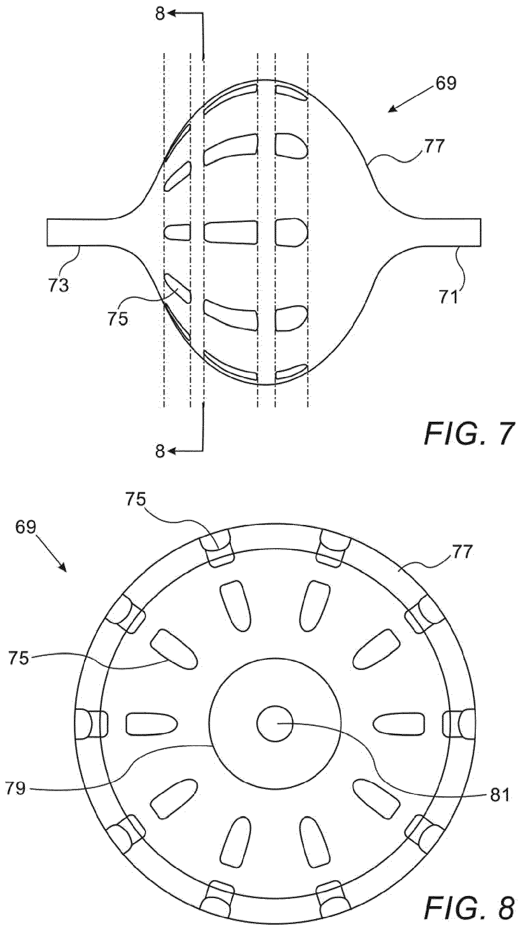

Reference is now made to Fig. 7, which is a side elevation of an embodiment of a balloon 69 having a proximal end 71 and a distal end 73 in accordance with an embodiment of the invention. An electrode assembly 75 is adhered to the exterior of the outer wall 77 of the balloon 69. At its proximal end 71, the balloon 69 is narrowed and configured to adapt to a connecting tube, which provides mechanical support and a supply of fluid. The distal end 73 is narrowed to permit fluid continuity between the interior of the balloon 69 and the lumen of a vessel.

Reference is now made to Fig. 8, which is a cut-away sectional view through line 8-8 of the balloon 69 (fig. 7) in accordance with an embodiment of the invention. A rim 79 seals the balloon 69 to a support (not shown), and prevents escape of fluid used for inflation of the balloon and irrigation fluid. An inner passage 81 permits fluid communication between a vessel and a location outside the body. For example contrast material may be transmitted through the passage 81.

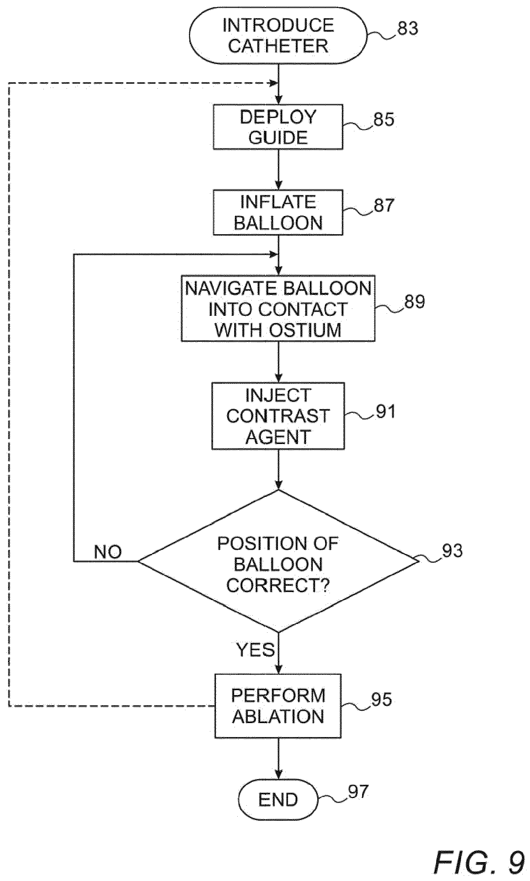

Reference is now made to Fig. 9, which is a flow-chart of a method of pulmonary vein isolation in accordance with an embodiment of the invention. At initial step 83 a cardiac catheter is conventionally introduced into the left atrium of a heart.

Next, at step 85 the lasso guide 41 is deployed and positioned to engage the interior wall of a pulmonary vein. Pre-ablation electrograms may be acquired once the lasso guide 41 is in position.

Next, at step 87 the balloon 47 is extended over the lasso guide 41 and inflated.

Next, at step 89 the balloon 47 is navigated into circumferential contact with a pulmonary vein ostium in order to occlude the ostium.

Next, at step 91 a radio-opaque contrast agent is injected through the lumen of the catheter, The contrast agent passes through a gap between the lasso guide 41 and the wall of the lumen in order to confirm that the balloon 47 is in a correct position against the pulmonary vein ostium. The contrast agent does not enter the balloon.

Control now proceeds to decision step 93, where it is determined if the balloon 47 is correctly positioned. If the determination at decision step 93 is negative, then control returns to step 89 and another attempt is made to position the balloon.

If the determination at decision step 93 is affirmative, then control proceeds to step 95 where ablation is performed using the ablation electrodes of the electrode assembly 53 (Fig. 4). A circumferential lesion is created in a region of tissue that circumscribes the pulmonary vein. The lesion blocks electrical propagation and effectively electrically isolates the pulmonary vein from the heart. Post-ablation electrograms may be obtained from the electrodes 43 of the lasso guide 41 (Fig. 2) in order to confirm functional isolation of the pulmonary vein.

After completion of the ablation, the procedure may be iterated using another pulmonary vein ostium by withdrawal of the balloon 47 and the lasso guide 41. Control may then return to step 85. Alternatively, the procedure may end by removal of the catheter 14 at final step 97.

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

## Claims

1. A method of ablation, comprising the steps of:
introducing a catheter into a left atrium of a heart, the catheter having a lumen and a distal end;
extending a lasso guide through the lumen of the catheter to engage an interior wall of a pulmonary vein;
deploying a balloon over a portion of the lasso guide, the balloon having a longitudinal axis and an electrode assembly disposed on an exterior wall thereof, the electrode assembly comprising a plurality of ablation electrodes circumferentially arranged about the longitudinal axis;
inflating the balloon;
positioning the balloon against the pulmonary vein at an ostium thereof, wherein the ablation electrodes are in galvanic contact with the pulmonary vein; and
conducting electrical energy through the ablation electrodes to produce a circumferential lesion that circumscribes the pulmonary vein.

2. The method according to claim 1, further comprising the step of after positioning the balloon injecting a contrast agent through the catheter into the pulmonary vein.

3. The method according to claim 1, further comprising the step of after positioning the balloon injecting a contrast agent through the catheter into the balloon.

4. The method according to claim 1, wherein the lasso guide has a mapping electrode disposed thereon, further comprising the step of obtaining a pre-ablation electrogram with the mapping electrode prior to performing the step of conducting electrical energy through the ablation electrodes.

5. The method according to claim 1, wherein the lasso guide has a mapping electrode disposed thereon, further comprising the step of obtaining a post-ablation electrogram with the mapping electrode after performing the step of conducting electrical energy through the ablation electrodes.

6. An ablation apparatus comprising:
a probe having a distal portion and a lumen;
a lasso guide that assumes a collapsed state for delivery through the lumen of the probe and an expanded state, the lasso guide having a plurality of mapping electrodes thereon, the mapping electrodes connectable to electrocardiographic circuitry;
an inflatable balloon deployable through the lumen over the lasso guide, the balloon having a longitudinal axis and an exterior wall; and
a plurality of ablation electrodes arranged circumferentially about the longitudinal axis on the exterior wall, the balloon being fenestrated by a plurality of irrigation pores and being connected to a source of fluid for passage through the pores.

7. The apparatus according to claim 6 further comprising a subassembly comprising a plurality of strips radiating outwardly from the longitudinal axis of the balloon wherein the ablation electrodes are disposed on the strips.

8. The apparatus according to claim 7, wherein the subassembly has apertures formed therethrough in, the apertures being in fluid communication with the pores of the balloon.

9. The apparatus according to claim 7, further comprising wires in the distal portion leading to the ablation electrodes wherein the strips of the subassembly comprise pigtails extending over a surface of the balloon and overlying the wires, respectively.
